# EUROPÄISCHE PATENTANMELDUNG

(11) **EP 3 960 049 A1**
(43) Veröffentlichungstag der Anmeldung: **02.03.2022**
(21) Anmeldenummer: 21191561.6
(22) Anmeldetag: 16.08.2021
(51) Int. Cl.: A47K 5/12, G08B 21/24

(54) **VORRICHTUNG ZUM ABGEBEN EINES FLUIDS, INSBESONDERE EINES REINIGUNGS-, PFLEGE-; ODER DESINFEKTIONSFLUIDS FÜR HÄNDE SOWIE SYSTEM ZUM ÜBERWACHEN EINER DERARTIGEN VORRICHTUNG**

(30) Priorität: 27.08.2020 DE 102020122427
(71) Anmelder: Huonker GmbH, 78052 Villingen-Schwenningen (DE)
(72) Erfinder: Huonker, Hans-Georg, 78052 Villingen-Schwenningen (DE)
(74) Vertreter: Westphal, Mussgnug & Partner Patentanwälte mbB

(57) **Zusammenfassung**

Die vorliegende Erfindung betrifft eine Vorrichtung (10) zum Abgeben eines Fluids, insbesondere eines Reinigungs-, Pflege-oder Desinfektionsfluids für Hände, umfassend ein Gehäuse (12), welches einen Hohlraum (14) umschließt, in welchen ein Behältnis (18) zum Vorhalten des Fluids einbringbar ist, und eine Gehäuseaußenoberfläche (28) bildet, eine Pump- und Dosiereinheit (20), welche mit dem Gehäuse (12) verbindbar oder verbunden ist, welche mit dem Behältnis (18) lösbar verbindbar ist, und mit welcher das Fluid aus dem Behältnis (18) heraus gefördert und abgegeben werden kann, eine Überwachungseinheit (54) umfasst, mit welcher Vorrichtungsbezogene und/oder nutzerbezogene Daten erfasst und gespeichert werden können, und ein mit der Überwachungseinheit (54) zusammenwirkendes Display (76) zum Eingeben und Anzeigen der nutzerbezogenen Daten und/oder der vorrichtungsbezogenen Daten. Ferner betrifft die Erfindung ein System zum Überwachen einer solchen Vorrichtung (10₂).

## Beschreibung

Die vorliegende Erfindung betrifft eine Vorrichtung zum Abgeben eines Fluids, insbesondere eines Reinigungs-, Pflege- oder Desinfektionsfluids für Hände. Weiterhin betrifft die Erfindung ein System zum Überwachen einer derartigen Vorrichtung.

Derartige Vorrichtungen sind beispielsweise aus der EP 3 650 128 A1 und der DE 10 58 272 A1 bekannt und werden auf vielfältige Weise angewendet. Beispielsweise in Handwerksbetrieben, bei denen die Handwerker regelmäßig ihre Hände verschmutzen, dienen derartige Vorrichtungen zum Abgeben eines Reinigungsfluids, insbesondere von Seife, um die Hände reinigen zu können. Da jedoch ein häufiges Händewaschen eine Belastung für die Haut im Bereich der Hände darstellt, werden derartige Vorrichtungen auch dazu verwendet, Pflegemittel, beispielsweise in Form von Handcreme, abzugeben. Hierdurch kann Hautkrankheiten vorgebeugt werden. Die Verwendung derartiger Pflegemittel kann für bestimmte Betriebe sogar vorgeschrieben sein.

Ein anderer, sehr wichtiger Anwendungsbereich derartiger Vorrichtungen findet sich in Einrichtungen des Gesundheitssektors, insbesondere in Krankenhäusern, Seniorenheimen und Arztpraxen. Dort werden die Vorrichtungen zum Abgeben von Desinfektionsfluiden verwendet, um die Verbreitung von Krankheitserregern wie Bakterien und Viren zu verringern.

Derartige Vorrichtungen weisen eine Pump- und Dosiereinheit auf, die in vielen Fällen mit einer Hand betätigt werden muss. Mit der anderen Hand wird das abgegebene Fluid aufgefangen, um es anschließend mit beiden Händen in die Haut im Bereich der Hände einzumassieren. Aufgrund der Tatsache, dass die Vorrichtungen von einer Vielzahl von Personen verwendet werden, besteht jedoch durch den Kontakt mit der Vorrichtung, der nötig ist, um die Pump- und Dosiereinheit zu betätigen, die Gefahr der Weitergabe von Krankheitserregern. Daher sind Vorrichtungen bekannt, bei denen die Pump- und Dosiereinheit mittels einer kontaktlos auslösbaren Antriebseinheit angetrieben werden kann, so dass kein Kontakt mit der Vorrichtung mehr notwendig ist. Hierdurch wird die Gefahr der Weitergabe von Krankheitserregern deutlich vermindert. Allerdings können derartige Vorrichtungen nicht garantieren, dass die Personen, die zur Desinfektion der Hände durch Richtlinien verpflichtet sind, die Hände auch wie vorgeschrieben desinfizieren. Zudem ist keine Dokumentation bezüglich der durchgeführten Handdesinfektionen möglich.

Aufgabe einer Ausführungsform der vorliegenden Erfindung ist es, eine Vorrichtung zum Abgeben eines Fluids, insbesondere eines Reinigungs-, Pflege- oder Desinfektionsfluids für Hände anzugeben, welche in der Lage ist, die durchgeführten Desinfektionsvorgänge zu dokumentieren. Darüber hinaus liegt einer Ausbildung der Erfindung die Aufgabe zugrunde, ein System zu schaffen, in welche die Vorrichtung zum Datenaustausch integriert werden kann.

Gelöst wird die Aufgabe mit den in den Ansprüchen 1 und 15 angegebenen Merkmalen. Vorteilhafte Weiterentwicklungen sind Gegenstand der Unteransprüche.

Eine Ausführungsform der Erfindung betrifft eine Vorrichtung zum Abgeben eines Fluids, insbesondere eines Reinigungs-, Pflege- oder Desinfektionsfluids für Hände, umfassend
- ein Gehäuse, welches
   o einen Hohlraum umschließt, in welchen ein Behältnis zum Vorhalten des Fluids einbringbar ist, und
   o eine Gehäuseaußenoberfläche bildet,
- eine Pump- und Dosiereinheit,
   o welche mit dem Gehäuse verbindbar oder verbunden ist,
   o welche mit dem Behältnis lösbar verbindbar ist, und
   o mit welcher das Fluid aus dem Behältnis heraus gefördert und abgegeben werden kann,
- eine Überwachungseinheit, mit welcher Vorrichtungsbezogene und/oder nutzerbezogene Daten erfasst und gespeichert werden können, und
- ein mit der Überwachungseinheit zusammenwirkendes Display zum Eingeben und Anzeigen der nutzerbezogenen Daten und/oder der vorrichtungsbezogenen Daten.

Die Überwachungseinheit steuert oder regelt sämtliche Vorgänge, welche die Vorrichtung ausführt. Mit der Überwachungseinheit kann beispielsweise erfasst werden, wann eine Abgabe von Fluid aus der Vorrichtung erfolgt ist. Diese Information ist ein Beispiel für vorrichtungsbezogene Daten. Darüber hinaus können mittels des Displays nutzerbezogene Daten eingegeben werden, welche einem Abgabevorgang zugeordnet werden können. Auf diese Weise kann eine Zuordnung des Abgabevorgangs zu einem bestimmten Benutzer der Vorrichtung erfolgen. Diese Zuordnung kann in der Überwachungseinheit gespeichert und für Dokumentationszwecke über eine entsprechende Schnittstelle ausgelesen werden. Somit ist es möglich, einen Nachweis darüber zu führen, ob eine bestimmte Person beispielsweise die vorgeschriebene Handdesinfektion tatsächlich auch durchgeführt hat.

Die Vorrichtung kann dabei mit einer mit der Überwachungseinheit zusammenwirkende Antriebseinheit ausgerüstet sein, mit welcher die Pump- und Dosiereinheit antreibbar ist. In diesem Fall kann die Abgabe des Fluids ohne die manuelle Betätigung eines Betätigungshebels ausgeführt werden. Zudem kann die Überwachungseinheit die abzugebende Fluidmenge vorgeben und protokollieren.

Nach Maßgabe einer weiteren Ausführungsform kann an der Gehäuseaußenoberfläche zumindest ein Ultraschallsensor angeordnet sein. Der Ultraschallsensor kann dabei derart an der Gehäuseoberfläche angeordnet sein, dass die von ihm ausgesendeten Ultraschallwellen die Hände eines Benutzers in einer für den Benutzer angenehmen Position erreichen können. Der Ultraschallsensor kann detektieren, ob der Benutzer Schmuck oder andere metallische Gegenstände an seinen Händen trägt, welche die Handdesinfektion stören könnten und daher gemäß vielen Vorschriften bei bestimmten Tätigkeiten nicht getragen werden dürfen. Die Vorrichtung kann für den Fall, dass der Benutzer einen oder mehrere metallische Gegenstände an den Händen trägt, ein Warnsignal, beispielsweise in optischer Form, welches auf dem Display angezeigt wird, ausgeben. Der eigentliche Abgabevorgang des Fluids wird erst dann freigegeben, wenn der Ultraschallsensor keine metallischen Gegenstände detektiert und folglich eine OK-Meldung vorliegt. Somit kann eine vorschriftsgemäße Handdesinfektion sichergestellt werden.

In einer weitergebildeten Ausführungsform kann an der Gehäuseaußenoberfläche eine Anzahl von Lichtquellen angeordnet sein, welche ultraviolette Strahlung emittieren. Dem Fluid können beispielsweise fluoreszierende Additive zugesetzt werden, welche in Wechselwirkung mit der ultravioletten Strahlung für den Benutzer sichtbar werden. Hält der Benutzer seine Händer unter die Lichtquellen, kann der Benutzer feststellen, ob die gesamte Handoberfläche vom Fluid, das von der Vorrichtung abgegeben worden ist, benetzt worden ist oder ob Stellen vorhanden sind, die vom Fluid nicht erreicht worden sind. Im letzteren Fall kann der Benutzer zusätzliches Fluid von der Vorrichtung anfordern und das zusätzliche Fluid auf die noch nicht benetzten Stellen auftragen. Hierdurch wird die Qualität der Handdesinfektion erhöht und die Gefahr der Verbreitung von Krankheitserregern verringert.

Bei einer weitergebildeten Ausführungsform kann die Vorrichtung zumindest einen UV-Sensor zum Registrieren der von den Händen eines Benutzers reflektierten ultravioletten Strahlen umfassen. Während für den Fall, dass die Vorrichtung keinen UV-Sensor (Sensor für die Erfassung von ultravioletter Strahlung) aufweist, die Kontrolle, ob das von der Vorrichtung abgegebene Fluid die gesamte Handoberfläche benetzt hat oder nicht, dem betreffenden Benutzer selbst unterliegt, kann diese Kontrolle mittels des UV-Sensors automatisiert werden, wozu auf der Überwachungseinheit eine entsprechende Auswertungssoftware hinterlegt ist. Die Qualität der Handdesinfektion wird hierdurch erhöht. Zudem kann automatisiert festgestellt werden, ob die betreffenden Vorschriften eingehalten worden sind oder nicht.

Bei einer weiteren Ausführungsform kann an der Gehäuseaußenoberfläche zumindest eine erste Kamera angeordnet sein. Die erste Kamera kann alternativ oder kumulativ zum UV-Sensor verwendet werden. Auch die erste Kamera kann zur Prüfung, ob die Handoberfläche vollständig vom Fluid benetzt worden ist, verwendet werden. Auf der Überwachungseinheit ist hierzu eine entsprechende Bildverarbeitungssoftware hinterlegt. Die Kamera kann alternativ oder kumulativ zum Ultraschallsensor verwendet werden. Die Bildverarbeitungssoftware kann so eingestellt werden, dass sie in der Lage ist, beispielsweise Schmuck oder Kunstnägel zu erkennen und entsprechende Warnsignale zu generieren. Die Qualität der Handdesinfektion wird hierdurch erhöht. Zudem kann automatisiert festgestellt werden, ob die betreffenden Vorschriften eingehalten worden sind oder nicht.

Eine weitergebildete Ausführungsform kann sich dadurch auszeichnen, dass die Vorrichtung eine Benutzererkennungseinrichtung aufweist, mit welcher die Identität des Benutzers der Vorrichtung festgestellt werden kann. Wie bereits erwähnt, ist es für Dokumentationszwecke und zum Nachweis, ob ein bestimmter Benutzer die vorgeschriebene Handdesinfektion durchgeführt hat, notwendig, einen bestimmten Abgabevorgang des Fluids einem Benutzer der betreffenden Vorrichtung zuzuordnen. Dies kann mittels einer Eingabe unter Verwendung des Displays geschehen. Mit der Benutzererkennungseinrichtung kann aber die Feststellung der Identität des Benutzers vereinfacht und verkürzt werden. Zudem kann die Benutzererkennungseinrichtung so ausgestaltet sein, dass ein Kontakt beispielsweise mit dem Display nicht notwendig ist, wodurch der Übertragung von Krankheitserregern entgegengewirkt wird.

Nach Maßgabe einer weiteren Ausführungsform kann die Benutzererkennungseinrichtung eine Ausleseeinheit zum Auslesen eines RFID-Transponders aufweisen. RFID-Transponder sind sehr handlich und können beispielsweise am Schlüsselanhänger oder an einem Armband mitgeführt werden. Häufig werden RFID-Transponder zur Zutrittskontrolle verwendet, so dass die Mitarbeiter beispielsweise eines Krankenhauses derartige RFID-Transponder ohnehin mitführen. Man muss den RFID-Transponder nur gegen die Ausleseeinheit halten, um die Identität des Benutzers feststellen zu können, was ein sehr schneller und einfach durchzuführender Vorgang ist.

Bei einer weiteren Ausführungsform kann es sich anbieten, dass die Benutzererkennungseinrichtung eine zweite Kamera umfasst. Im Gegensatz zu RFID-Transponder prüft die zweite Kamera biometrische Merkmale eines Benutzers, beispielsweise die Gesichtszüge oder die Maserung der Iris. Während RFID-Transponder zwischen mehreren Benutzern ausgetauscht werden können und somit eine Manipulation der Identität des Benutzers möglich ist, ist eine Manipulation bei der Verwendung von biometrischen Merkmalen zumindest deutlich schwieriger.

In einer weitergebildeten Ausführungsform können die Ausleseeinheit und/oder die zweite Kamera im Display angeordnet sein. Hierdurch kann der Montageaufwand der Vorrichtung reduziert werden, da insbesondere die Verkabelung im Vergleich zu einer Ausführungsform vereinfacht wird, bei welcher die Ausleseeinheit und die zweite Kamera außerhalb des Displays angeordnet sind. Da sich der Benutzer intuitiv am Display orientieren wird, wird ein langes Suchen nach der Ausleseeinheit oder der zweiten Kamera vermieden, wodurch die Aufenthaltsdauer an der Vorrichtung auch für Benutzer verringert wird, welche die Vorrichtung erstmalig oder nur sehr selten verwendet, beispielsweise Besucher.

Bei einer weitergebildeten Ausführungsform kann im Display eine Anzahl von Tastenfeldern angeordnet sein, wobei jedes Tastenfeld einer bestimmten, mit einem Desinfektionsvorgang in Verbindung stehenden Tätigkeit zugeordnet ist. Beispielsweise in einem Krankenhaus können die dort geltenden Vorschriften unterschiedliche Standards zur Handdesinfektion vorschreiben. Beispielsweise ist eine hygienische Handdesinfektion anders durchzuführen als eine chirurgische Handdesinfektion. So kann für eine Tätigkeit eine hygienische Handdesinfektion, beispielsweise für einen Katheterwechsel, und für eine andere Tätigkeit, beispielsweise ein operativer Eingriff, eine chirurgische Handdesinfektion vorgeschrieben sein. Die von der Vorrichtung abgegebene Menge des Fluids kann sich dabei von einer hygienischen Handdesinfektion unterscheiden.

Folglich kann ein Benutzer, der eine bestimmte Tätigkeit durchführen will, ein dieser Tätigkeit zugeordnetes Tastenfeld wählen und anschließend die Handdesinfektion durchführen. Die von der Vorrichtung abgegebene Menge des Fluids kann sich je nach Tätigkeit unterscheiden. Die Überwachungseinheit kann entsprechend konfiguriert sein. Somit kann sichergestellt werden, dass für die betreffende Handdesinfektion die passende Menge des Fluids abgegeben wird, wodurch eine Über- und Unterdosierung vermieden wird. Gleichzeitig kann nachvollzogen werden, welche Tätigkeit welche Person mit der hierzu passenden Handdesinfektion wann durchgeführt hat. Compliance-Vorschriften können hierdurch besonders einfach erfüllt und dokumentiert werden.

Bei einer weiteren Ausführungsform kann die Vorrichtung eine Sende- und Empfangseinheit zum Datenaustausch mit externen Einheiten umfassen. Wie erwähnt, können vorrichtungsbezogene und/oder nutzerbezogene Daten mit der Überwachungseinheit gespeichert werden. Um diese Daten jedoch weiter zu bearbeiten, muss die Überwachungseinheit ausgelesen werden. Hierzu kann beispielsweise ein entsprechend eingerichtetes Auslesegerät verwendet werden, welches an die Vorrichtung angeschlossen werden muss. In der vorliegenden Ausführungsform jedoch umfasst die Vorrichtung eine Sende- und Empfangseinheit, mit welcher die gespeicherten Daten drahtlos, beispielsweise über Bluetooth oder WLAN, an eine externe Einheit übermittelt werden kann. Der Datenaustausch kann in regelmäßigen Intervallen oder quasikontinuierlich erfolgen. Im letzten Fall kann der Zustand der Vorrichtung nahezu in Echtzeit ermittelt werden. Außergewöhnliche Ereignisse, die bestimmte Maßnahmen erforderlich machen, können somit zeitnah eingeleitet werden, wodurch ein längerer Stillstand der Vorrichtung verhindert wird.

Eine weitergebildete Ausführungsform kann sich dadurch auszeichnen, dass die Vorrichtung
- einen Füllstandssensor zum Bestimmen des Füllstands des Fluids im Hohlraum und/oder
- einen Temperatursensor zum Bestimmen der Temperatur des Fluids im Hohlraum umfasst.

Die Ermittlung des Füllstands ermöglicht es, ein entsprechendes Signal zu erzeugen, wenn der Füllstand ein gewisses Maß unterschreitet. Hierdurch kann der Austausch des Behältnisses durch ein volles Behältnis rechtzeitig veranlasst werden, so dass verhindert wird, dass das Behältnis leer ist und die Vorrichtung nicht bestimmungsgemäß verwendet werden kann.

Im Normalfall kann davon ausgegangen werden, dass das Fluid im Behältnis in etwa dieselbe Temperatur aufweist wie die Umgebung der Vorrichtung. Steigt jedoch die Temperatur des Fluids an, kann dies auf eine Verkeimung des Fluids hindeuten, wodurch das Fluid seinen bestimmungsgemäßen Zweck nicht mehr erfüllen kann und ausgetauscht werden muss. Insofern kann hierdurch ein signifikanter Beitrag zur Vermeidung der Verbreitung von Krankheitserregern erzielt werden. Darüber hinaus kann der Temperatursensor auch dazu verwendet werden, Brandherde auf zu spüren und deren Position zu ermitteln. Der Anstieg der Temperatur des Fluids infolge einer Verkeimung ist deutlich langsamer als eine Temperaturerhöhung infolge eines Brandes, zudem ist der Temperaturanstieg im Falle eines Brandes deutlich stärker ausgeprägt als in Folge einer Verkeimung. Eine Unterscheidung zwischen einem Brand und einer Verkeimung ist daher mit ausreichender Sicherheit möglich.

Nach Maßgabe einer weiteren Ausführungsform kann die Antriebseinheit mit einem berührungslos auslösbaren Bewegungssensor aktivierbar sein und der Bewegungssensor als ein Ultraschallsensor oder als ein Näherungssensor ausgestaltet sein. Bewegungssensoren, die üblicherweise für Vorrichtungen der vorliegenden Art eingesetzt werden, sind nach Art von Lichtschranken ausgebildet. Es kann jedoch sein, dass bei ungünstigen Lichtbedingungen keine zuverlässige Betätigung der Antriebseinheit mehr möglich ist. Die Verwendung eines Ultraschallsensors erhöht die Zuverlässigkeit der Aktivierung der Antriebseinheit. Aber auch die Näherungssensoren können so ausgestaltet sein, dass sie bei ungünstigen Lichtbedingungen verlässlich auslösen, insbesondere dann, wenn sie als induktive oder kapazitive Näherungssensoren ausgestaltet sind.

Bei einer weiteren Ausführungsform kann es sich anbieten, dass die Gehäuseaußenoberfläche als eine antibakterielle Oberfläche und/oder als eine superhydrophobe Oberfläche und/oder als eine superamphiphobe Oberfläche ausgebildet ist. Das Wachstum von Mikroorganismen wie Bakterien und Pilzkulturen stellt ein Hygienerisiko dar. Viele der Mikroorganismen sind Krankheitserreger. Eine antibakterielle Oberfläche verhindert das Wachstum von Mikroorganismen. Eine antibakterielle Oberfläche kann beispielsweise mit einer auf die Gehäuseoberfläche aufgebrachten Beschichtung bereitgestellt werden. In dieser Beschichtung können Additive enthalten sein, welche gegenüber Mikroorganismen hoch wirksam, für den menschlichen Organismus aber unbedenklich sind. Diese Additive müssen aber nicht zwangsläufig in einer auf die Gehäuseoberfläche aufgebrachten Beschichtung enthalten sein, sondern können alternativ dem Material, aus dem das Gehäuse gefertigt ist, zugesetzt werden. Auch ist es möglich, das komplette Gehäuse aus einem antibakteriellen Material zu fertigen.

Die superhydrophobe Oberfläche verhindert das Anhaften von Mikroorganismen, insbesondere von Krankheitserregern, wodurch die Gefahr der Verbreitung von Krankheitserregern beim Gebrauch der vorschlagsgemäßen Vorrichtung deutlich verringert wird. Die Eigenschaft, dass ein Anhaften von Krankheitserregern und anderen Partikeln an den superhydrophobe Oberflächen verhindert wird, wird häufig auch als Lotus-Effekt bezeichnet. Die superhydrophobe Oberfläche kann mit einer auf die Gehäuseoberfläche aufgebrachte Beschichtung bereitgestellt werden. Diese Beschichtung kann auch die bereits erwähnten antibakteriellen Additive enthalten. Eine super-amphiphobe Oberfläche ist sowohl wasser- als auch ölabweisend und kann ebenfalls als eine Beschichtung auf die Gehäuseoberfläche aufgebracht werden.

Eine Ausbildung der Erfindung betrifft ein System zum Überwachen einer Vorrichtung nach einer der vorherigen Ausführungsformen, umfassend
- eine Vorrichtung nach einer der vorherigen Ausführungsformen,
- eine erste externe Einheit, auf welcher nutzerbezogene Daten gespeichert sind, die mittels der Sende- und Empfangseinheit auslesbar sind, und
- eine zweite externe Einheit, auf welcher die nutzerbezogenen Daten und vorrichtungsbezogene Daten ablegbar und auswertbar sind, wobei die zweite externe Einheit eine zweite Sende- und Empfangseinheit zum Datenaustausch mit der Sende- und Empfangseinheit der Vorrichtung aufweist.

Die technischen Effekte und Vorteile, die sich mit dem vorschlagsgemäßen System erreichen lassen, entsprechen denjenigen, die für die betreffende Ausführungsform der Vorrichtung erörtert worden sind. Zusammenfassend sei darauf hingewiesen, dass ein Datenaustausch auf einfache Art und Weise möglich ist. Mit der zweiten externen Einheit können die Daten, welche auf der Überwachungseinheit gespeichert sind, ausgewertet werden. Somit lässt sich beispielsweise feststellen, wer wann die Vorrichtung benutzt hat. Hieraus wiederum lässt sich feststellen, ob die betreffende Person bestimmte Vorschriften eingehalten hat oder nicht. Es lässt sich daher eine umfangreiche Dokumentation bezüglich der Benutzung der Vorrichtung realisieren. Darüber hinaus lässt sich auch feststellen und dokumentieren, wenn beispielsweise die Temperatur des Fluids einen bestimmten Wert überschritten oder der Füllstand des Fluids einen bestimmten Wert unterschritten hat. Geeignete Gegenmaßnahmen können somit zeitnah eingeleitet und ein Stillstand der Vorrichtung vermieden oder gering gehalten werden.

Das System kann ferner so betrieben werden, dass der Zugang beispielsweise zu einem Operationssaal nur dann freigegeben wird, wenn eine grundsätzlich zum Zutritt berechtigte Person ihre Hände vorschriftsmäßig desinfiziert hat. Hierzu kann das System mit einem Türöffner zusammenwirken, der im Falle einer vorschriftsmäßigen Handdesinfektion aktiviert wird und die betreffende Tür öffnet.

Bei einer weiteren Ausbildung kann vorgesehen sein, dass die zweite Sende- und Empfangseinheit derart eingerichtet ist, dass ein Datenaustausch mit einer dritten externen Einheit ermöglicht wird. Die zweite externe Einheit kann beispielsweise ein Server eines Krankenhauses, einer Arztpraxis oder eines Betriebs sein. Die dritte externe Einheit kann ein übergeordneter Server sein, wo Daten von vielen zweiten externen Einheiten zusammenlaufen. Hierdurch lassen sich Infektionsketten nachvollziehen und die betreffenden Benutzer entsprechend informieren. Wenn beispielsweise festgestellt worden ist, dass sich eine Person mit einem bestimmten Erreger infiziert hat und diese Person eine der vorschlagsgemäßen Vorrichtungen verwendet hat, kann eine entsprechende Warnung an die Personen, welche die Vorrichtung kurz danach verwendet haben, geschickt werden. Diese Personen können dann entsprechende Gegenmaßnahmen wie Quarantäne oder dergleichen ergreifen. Zudem kann die betreffende Vorrichtung einer gründlichen Desinfektion beispielsweise durch Autoklavieren oder einer Reinigung in einer Spülmaschine unterzogen werden. Die verwendeten Kunststoffe weisen eine ausreichende Widerstandsfähigkeit gegenüber den beim Reinigen in einer Spülmaschine oder beim Autoklavieren herrschenden Bedingungen auf. Auch hierdurch kann der Verbreitung von Krankheitserregern entgegengesteuert werden.

Beispielhafte Ausführungsformen der Erfindung werden im Folgenden unter Bezugnahme auf die beigefügten Zeichnungen näher erläutert. Es zeigen
- Figur 1: eine perspektivische Ansicht einer ersten Ausführungsform einer vorschlagsgemäßen Vorrichtung zum Abgeben eines Fluids, insbesondere eines Reinigungs-, Pflege- oder Desinfektionsfluids für Hände, mit Verschlusskörper,
- Figur 2: die in Figur 1 gezeigte Vorrichtung anhand einer Seitenansicht mit Verschlusskörper,
- Figur 3A: eine perspektivische Schnittdarstellung durch die in Figur 1 gezeigte Vorrichtung,
- Figur 3B: eine isolierte Darstellung einer Pump- und Dosiereinheit,
- Figur 4: eine perspektivische Schnittdarstellung durch eine zweiten Ausführungsform der vorschlagsgemäßen Vorrichtung in einer ersten Konfiguration, und
- Figur 5: eine perspektivische Schnittdarstellung durch die zweite Ausführungsform der vorschlagsgemäßen Vorrichtung in einer zweiten Konfiguration, und
- Figur 6: eine prinzipielle Darstellung eines Systems zum Überwachen der in den Figuren 1 bis 3 gezeigten Vorrichtung,
- Figur 7: eine erste perspektivische Darstellung einer dritten Ausführungsform der vorschlagsgemäßen Vorrichtung, und
- Figur 8: eine zweite perspektivische Darstellung der dritten Ausführungsform der vorschlagsgemäßen Vorrichtung.

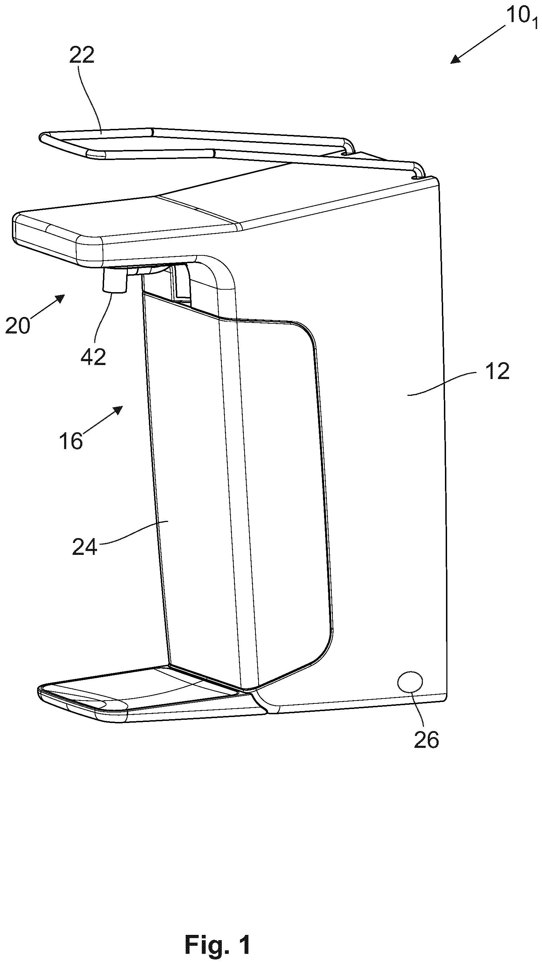

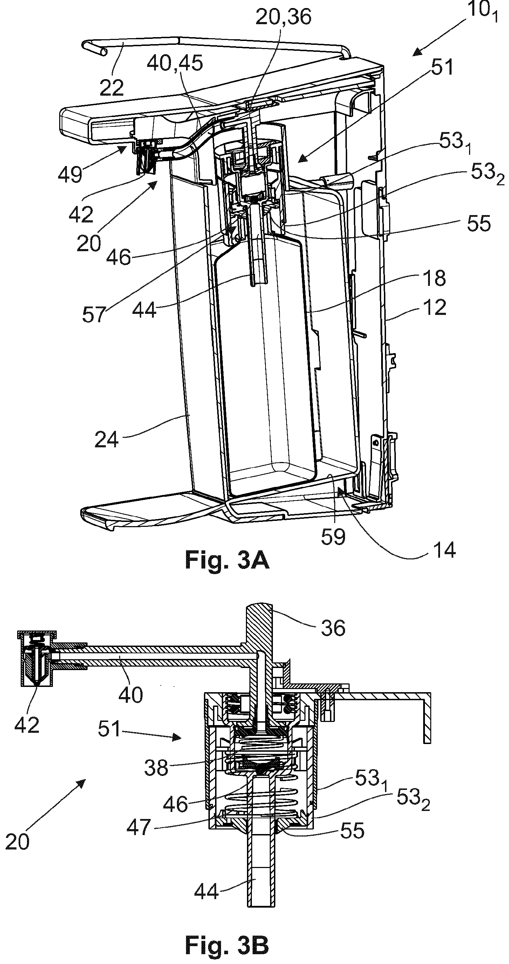

Figur 1 zeigt eine perspektivische Ansicht einer ersten Ausführungsform einer vorschlagsgemäßen Vorrichtung 10₁ zum Abgeben eines Fluids, insbesondere eines Reinigungs-, Pflege- oder Desinfektionsfluids für Hände. Die Vorrichtung 10₁ umfasst ein Gehäuse 12, welches einen Hohlraum 14 umschließt (siehe Figur 3A). Das Gehäuse 12 bildet eine Öffnung 16, durch welche der Hohlraum 14 zugänglich ist. Wie in Figur 3A zu sehen, kann ein Behältnis 18, in welchem ein Fluid, beispielsweise ein Reinigungsfluid, ein Pflegefluid oder ein Desinfektionsfluid, aufbewahrt werden kann, in den Hohlraum 14 eingebracht und mit einer Pump- und Dosiereinheit 20, welche nachfolgend noch genauer beschrieben wird, verbunden werden, beispielsweise mittels eines Gewindes oder eines Bajonettverschlusses (nicht dargestellt). In Figur 1 ist ein Betätigungshebel 22 der Pumpund Dosiereinheit 20 erkennbar, der drehbar am Gehäuse 12 befestigt ist.

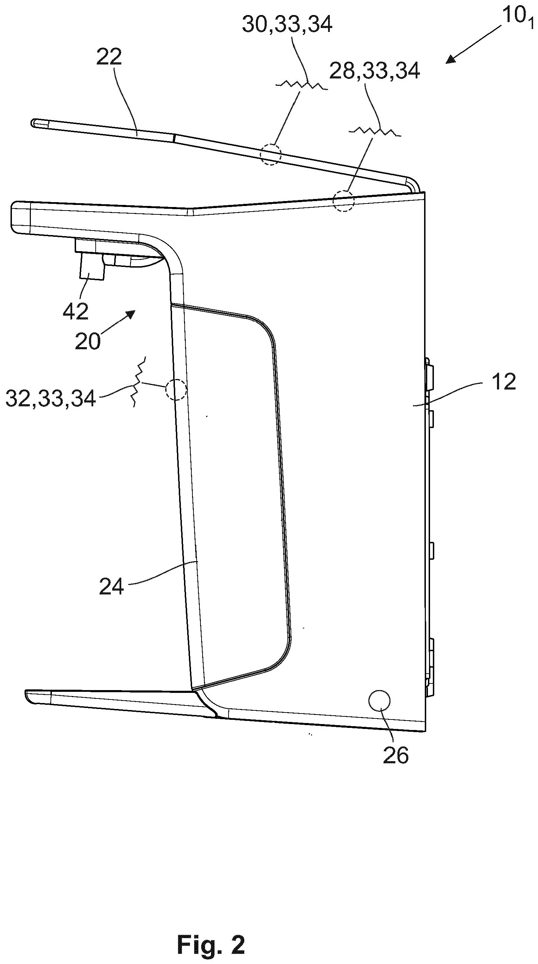

In Figur 2 ist die in Figur 1 gezeigte Vorrichtung 10₁ anhand einer Seitenansicht gezeigt. Sowohl aus Figur 1 als auch aus Figur 2 erkennt man, dass die Öffnung 16 mittels eines Verschlusskörpers 24 verschlossen ist, weshalb das Behältnis 18 in den Figuren 1 und 2 nicht mehr erkennbar ist. Es kann aber vorgeschrieben sein, dass das Behältnis 18 von außen sichtbar ist. In diesem Fall kann der Verschlusskörper 24 aus einem transparenten Material gefertigt sein. Der Verschlusskörper 24 ist mittels einer Steckverbindung mit dem Gehäuse 12 verrastbar. Zum Lösen vom Gehäuse 12 muss der Verschlusskörper 24 von einem Benutzer etwas zusammengedrückt werden. Nicht dargestellt ist eine Ausführungsform, in welcher der Verschlusskörper 24 zwischen einer Schließstellung und einer Offenstellung, bewegbar am Gehäuse 12 befestigt ist. Hierzu kann das Gehäuse 12 eine Aufnahmeöffnung 26 aufweisen, wobei Details der Befestigung aus Darstellungsgründen nicht gezeigt sind.

Das Gehäuse 12 bildet eine Gehäuseaußenoberfläche 28 (siehe Figur 2). Entsprechend bildet die Pump- und Dosiereinheit 20 eine Pump- und Dosiereinheit-Außenoberfläche 30 und der Verschlusskörper 24 eine Verschlusskörper-Außenoberfläche 32. Im dargestellten Ausführungsbeispiel wird die Pump- und Dosiereinheit-Außenoberfläche 30 vom bereits erwähnten Betätigungshebel 22 der Pump- und Dosiereinheit 20 gebildet. Die Gehäuseaußenoberfläche 28, die Pump- und Dosiereinheit-Außenoberfläche 30 und die Verschlusskörper-Außenoberfläche 32 sind jeweils als eine antibakterielle Oberfläche 33 und als eine superhydrophobe Oberfläche 34 ausgebildet, welche in Figur 2 symbolisch und vergrößert dargestellt sind. Die antibakterielle Oberfläche 33 verhindert das Wachstum von Mikroorganismen und die superhydrophobe Oberfläche 34 verhindert das Anhaften von Partikeln, insbesondere von Krankheitserregern, wodurch der Verbreitung von Krankheitserregern und der von ihnen hervorgerufenen Krankheiten entgegengewirkt werden kann. Der Betätigungshebel 22 kann alternativ aus Edelstahl gefertigt sein, ohne dass er eine Beschichtung aufweist.

Wie aus Figur 2 erkennbar, schließt die Verschlusskörper-Außenoberfläche 32 versatzfrei mit der Gehäuseaußenoberfläche 28 ab, wenn der Verschlusskörper 24 mit dem Gehäuse 12 verbunden ist. Die Ausbildung von Toträumen, in welchen sich Krankheitserreger ansammeln und vermehren könnten, wird hierdurch vermieden.

Figur 3A zeigt eine perspektivische Schnittdarstellung durch die in den Figuren 1 und 2 dargestellte Vorrichtung 10₁. Zur Verdeutlichung des Aufbaus der Pump- und Dosiereinheit 20 ist diese in einer etwas anderen Ausführungsform separat in Figur 3B gezeigt, wobei die im Folgenden beschriebenen Funktionen bei beiden Ausführungsformen gleich ist.

Man erkennt, dass die Pump- und Dosiereinheit 20 einen Pumpenstempel 36 aufweist, der mit dem Betätigungshebel 22 in den Hohlraum 14 des Gehäuses 12 hineingedrückt werden kann, wobei der Pumpenstempel 36 mit einer ersten Feder 38 vorgespannt ist. Der Pumpenstempel 36 umfasst einen Kanal 40, der an seinem freien Ende eine Abgabeöffnung 42 bildet, die als eine Schaumdüse ausgeführt sein kann. An seinem geschlossenen Ende mündet der Kanal 40 in ein Saugrohr 44, welches in das Behältnis 18 hineinragt, wenn das Behältnis 18 mit der Pump- und Dosiereinheit 20 verbunden ist. An das Saugrohr 44 kann ein nicht dargestellter Verlängerungsschlauch angeschlossen werden, der bis zum Grund des Behältnisses 18 reicht. Zwischen der Abgabeöffnung 42 und dem Kopf des Pumpenstempels 36 wird der Kanal 40 in der in Figur 3A gezeigten Ausführungsform von einem flexiblen Schlauch 45 gebildet, damit Relativbewegungen zwischen dem Pumpenstempel 36 und der Abgabeöffnung 42 ausgeglichen werden können. Darüber hinaus ist die Pump- und Dosiereinheit 20 mit einem Rückschlagventil 46 versehen, welches mit der ersten Feder 38 in eine Schließstellung, wie in den Figuren 3A und 3B gezeigt, vorgespannt wird.

Wenn ein Benutzer auf den Betätigungshebel 22 drückt, wird der Pumpenstempel 36 in den Hohlraum 14 des Gehäuses 12 verschoben, wobei die erste Feder 38 komprimiert wird. Das Rückschlagventil 46 verbleibt in der Schließstellung. Aufgrund der Verkleinerung des Volumens zwischen der Abgabeöffnung 42 und dem Rückschlagventil 46 tritt das Fluid durch die Abgabeöffnung 42 aus, sofern der Kanal 40 zwischen dem Rückschlagventil 46 und der Abgabeöffnung 42 vollständig mit Fluid gefüllt ist.

Lässt der Benutzer den Betätigungshebel 22 los, so wird der Pumpenstempel 36 zusammen mit dem Betätigungshebel 22 von der ersten Feder 38 wieder zurück in die Ausgangsstellung gestellt. Aufgrund der Vergrößerung des Volumens zwischen dem Rückschlagventil 46 und der Abgabeöffnung 42 wird das Rückschlagventil 46 geöffnet und das Fluid aus dem Behältnis 18 in das Saugrohr 44 gesaugt. Um zu verhindern, dass bereits abgegebenes Fluid oder Luft aus der Umgebung durch die Abgabeöffnung 42 zurück in den Kanal 40 gesaugt wird, ist in der Abgabeöffnung 42 eine Rückschlagventil-Funktion integriert.

Aus Figur 3A ist ebenfalls zu entnehmen, dass die Pump- und Dosiereinheit 30 mittels einer Befestigungseinrichtung 49 lösbar mit dem Gehäuse 12 verbunden ist. Insbesondere im Bereich der Abgabeöffnung 42 ist erkennbar, dass die Befestigungseinrichtung 49 nutenförmige Abschnitte aufweist, in welche die Pump- und Dosiereinheit 30 mit einer translatorischen Bewegung senkrecht zur Schnittebene der Figur 3A eingeschoben und aus dieser wieder entfernt werden kann.

Ferner verfügt die Pump- und Dosiereinheit 30 über eine Adaptereinheit 51, welche in der vorliegenden Ausführungsform einen ersten rohrförmigen Abschnitt 53₁ und einen zweiten rohrförmigen Abschnitt 53₂ umfasst, die ineinander eingreifen und mittels eines Gewindes miteinander befestigt sind (siehe insbesondere Figur 3B). Durch Drehen kann der zweite rohrförmige Abschnitt 53₂ mehr oder weniger weit in den ersten rohrförmigen Abschnitt 53₁ eingebracht werden, infolgedessen der zweite rohrförmige Abschnitt 53₂ mehr oder weniger weit aus dem ersten rohrförmigen Abschnitt 53₁ heraussteht. Im zweiten rohrförmigen Abschnitt 53₂ ist ein Dichtkörper 55 axial bewegbar gelagert, wobei der Dichtkörper 55 mit einer zweiten Feder 47 in die in Figur 3B gezeigte Stellung vorgespannt wird.

Zum Einsetzen des Behältnisses 18 wird dieses mit einer Öffnung 57 gegen den Dichtkörper 55 gedrückt und mit dem Boden auf eine geneigte Auflagefläche 59 aufgelegt (siehe Figur 3A). Je nach Größe des Behältnisses 18 wird der Dichtkörper 55 mehr oder weniger weit zum Rückschlagventil 46 hin verschoben, wobei die zweite Feder 47 komprimiert wird. Hierdurch können einerseits Behältnisse 18 verwendet werden, welche sich in ihrer Höhe in gewissen Grenzen unterscheiden. Andererseits wird dafür gesorgt, dass ein ausreichend großer Anpressdruck zwischen dem Dichtkörper 55 und der Öffnung 57 des Behältnisses 18 wirkt, um das Behältnis 18 gegenüber der Pump- und Dosiereinheit 20 abzudichten.

Wenn der Höhenunterschied zwischen zwei Behältnissen 18 den Weg, welchen der Dichtkörper 55 innerhalb des rohrförmiges Abschnitts 53₂ zurücklegen kann, übersteigt, kann der zweite rohrförmige Abschnitt 53₂ durch Drehen weiter aus dem ersten rohrförmigen Abschnitt 53₁ heraus oder in diesen hinein bewegt werden. Dabei wird angestrebt, dass das freie Ende des zweiten rohrförmigen Abschnitts 53₂ mit dem Behältnis 18 zur Anlage kommt, um die Öffnung 57 des Behältnisses 18 vor dem Eindringen von Schmutz zu schützen.

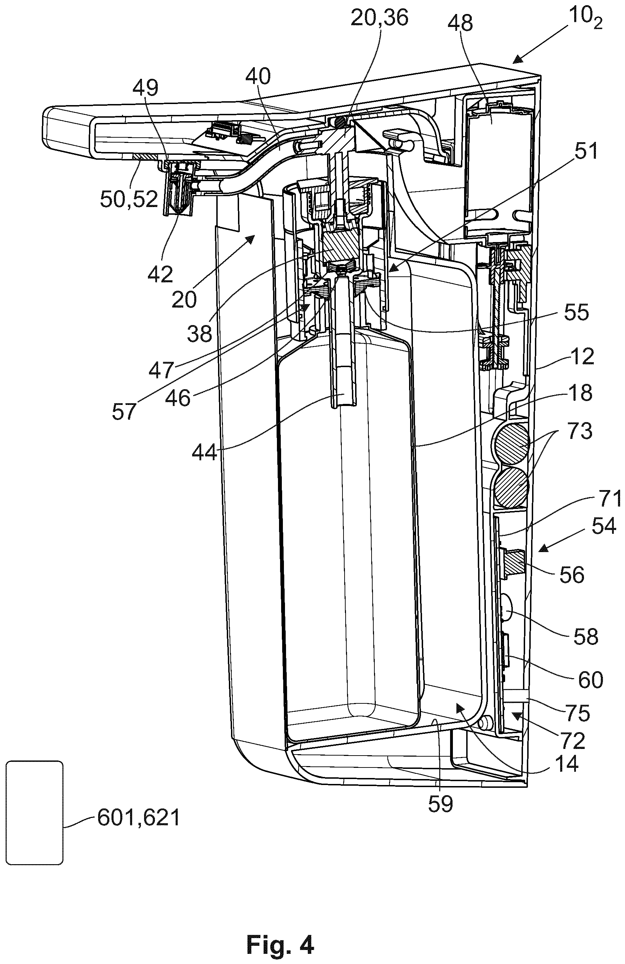

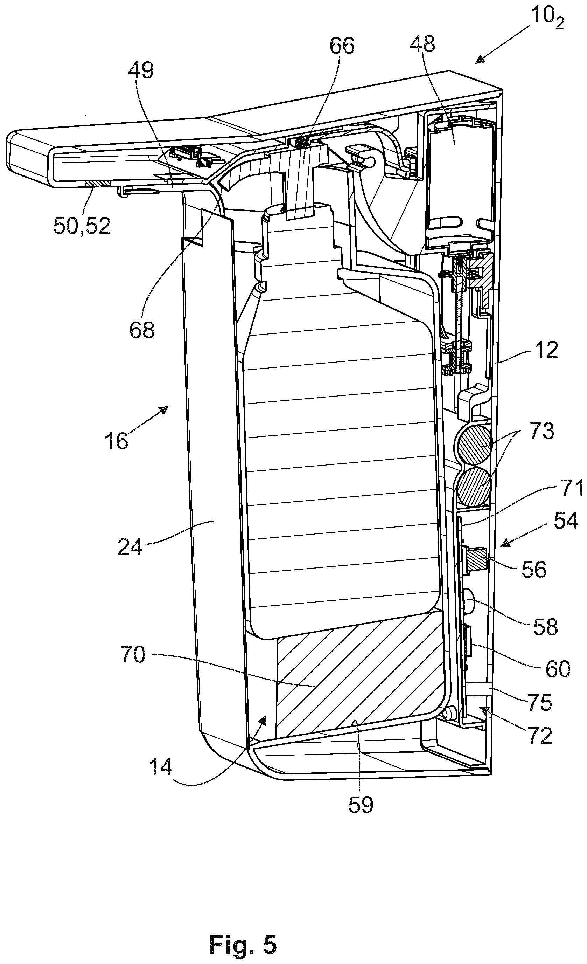

In Figur 4 ist eine zweite Ausführungsform der Vorrichtung 10₂ anhand einer perspektivischen Schnittdarstellung gezeigt, welche sich an die Darstellung der Figur 3 anlehnt. Die Vorrichtung 10₂ befindet sich in einer ersten Konfiguration. Figur 5 zeigt die zweite Ausführungsform der Vorrichtung 10₂ ebenfalls anhand einer perspektivischen Schnittdarstellung, wobei sich die Vorrichtung 10₂ in einer zweiten Konfiguration befindet. Auf die beiden Konfigurationen wird später noch eingegangen. Aus Figur 4 ist erkennbar, dass die Vorrichtung 10₂ mit einer Antriebseinheit 48 ausgestattet ist, mit welcher der Pumpenstempel 36 unabhängig in den Hohlraum 14 hinein bewegt werden kann und welche beispielsweise als ein Elektromotor ausgebildet sein kann. In diesem Fall ist es nicht notwendig, dass der Benutzer mit seiner Hand auf den Betätigungshebel 22 drückt. Folglich weist die Vorrichtung 10₂ nach der zweiten Ausführungsform keinen Betätigungshebel 22 auf.

Zum Aktivieren der Antriebseinheit 48 umfasst die Vorrichtung 10₂ einen Bewegungssensor 50, welcher im vorliegenden Ausführungsbeispiel als ein Ultraschallsensor 52 oder ein Näherungssensor 52 ausgeführt ist. Der Näherungssensor 50 ist in der Figur 4 nur prinzipiell dargestellt und ist auf einer hier nicht näher dargestellten Sensorplatine angeordnet, die im Hohlraum über dem Näherungssensor 50 der Vorrichtung 10₂ angeordnet ist. Der Benutzer muss seine Hand in den Erfassungsbereich des Bewegungssensors 50 einbringen, ohne jedoch dabei die Vorrichtung 10₂ berühren zu müssen. Infolgedessen wird vom Bewegungssensor 50 ein Signal erzeugt und an die Antriebseinheit 48 weitergeleitet. Die Antriebseinheit wird aktiviert und eine bestimmte Menge von Fluid über die Abgabeöffnung 42 abgegeben. Die Wirkweise der Pump- und Dosiereinheit 20 entspricht dabei derjenigen, die für die erste Ausführungsform der Vorrichtung 10₁ beschrieben worden ist mit dem wesentlichen Unterschied, dass die Bewegung des Pumpenstempels 36 von der Antriebseinheit 48 und nicht infolge des Bewegens des Betätigungshebels 22 bewirkt wird.

Da die Vorrichtung 10₂ nach der zweiten Ausführungsform kontaktlos aktiviert werden kann, wird die Gefahr, dass sich Partikel, insbesondere Krankheitserreger, die sich an der Pumpund Dosiereinheit-Außenoberfläche 30 angelagert haben, an der Hand des Benutzers anlagern und hierdurch in den Körper des Benutzers gelangen können.

Wie man ebenfalls aus Figur 4 erkennen kann, ist die Vorrichtung 10₂ mit einer Überwachungseinheit 54 ausgerüstet, mit welcher vorrichtungsbezogene und/oder benutzerbezogene Daten erfasst und gespeichert werden können. Die Überwachungseinheit 54 umfasst eine Platine 71, die in einem vom Gehäuse 12 umschlossenen weiteren Hohlraum 72 angeordnet ist. In Figur 4 ist ferner erkennbar, dass die Vorrichtung 102 einen auf der Platine 71 angeordneten Füllstandssensor 56 aufweist, mit welchem der Füllstand des Fluids im Behältnis 18 ermittelt werden kann. Darüber hinaus ist die Vorrichtung 10₂ mit einem Temperatursensor 58 ausgerüstet, der ebenfalls auf der Platine 71 angeordnet ist und mit welchem die Temperatur im Umfeld des Temperatursensors 58 bestimmt werden kann. Der Temperatursensor 58 ist so angeordnet, dass er im Wesentlichen die Temperatur des Fluids im Behältnis 18 bestimmt. Wenn jedoch die Temperatur im Umfeld der Vorrichtung 101 deutlich sinkt oder ansteigt, wie es beispielsweise bei einem Brand der Fall sein kann, wird der Temperatursensor 58 diese Temperaturänderung ebenfalls erfassen.

Sowohl der Füllstandssensor 56 als auch der Temperatursensor 58 wirken mit der Überwachungseinheit 54 zusammen und liefern zumindest einen Teil der bereits erwähnten vorrichtungsbezogenen Daten.

Aus Figur 4 ist auch erkennbar, dass die Vorrichtung 10₂ darüber hinaus über eine Sende- und Empfangseinheit 60 verfügt, die auch auf der Platine 71 angeordnet ist. Mit dieser Sendeund Empfangseinheit 60 können Daten, welche auf der Überwachungseinheit 54 abgelegt sind, mit externen Einheiten 62 ausgetauscht werden. Eine erste externe Einheit 621 ist in Figur 4 gezeigt, die beispielsweise als ein Smartphone, als ein Transponder, als ein Barcode oder der gleichen ausgeführt sein kann. Hierdurch kann ein Benutzer, der die Vorrichtung 10₂ verwendet, identifiziert werden. So ist es beispielsweise möglich, die Antriebseinheit 48 erst dann aktivieren zu können, wenn zuvor ein Benutzer als ein autorisierter Benutzer erkannt werden konnte.

Aus Figur 4 ist ferner erkennbar, dass die Vorrichtung 10₂ zwei Batterien 73 umfasst, mit welcher die Versorgung der Antriebseinheit 48 und der elektronischen Komponenten wie des Temperatursensors 58 mit elektrischer Energie gewährleistet werden kann. Wie erwähnt, verfügt die Vorrichtung 10₂ über eine Sendeund Empfangseinheit 60, mit welcher Daten drahtlos mit externen Einheiten 621 ausgetauscht werden können. Zusätzlich verfügt die Vorrichtung 10₂ auch über einen Ethernet-Anschluss 75, mit welchem die Vorrichtung 10₂ drahtgebunden an ein LAN angeschlossen werden kann. Bei einer entsprechenden Konfiguration des LAN können die elektronischen Komponenten auch über das LAN mit elektrischer Energie versorgt werden (Power over Ethernet).

Wie erwähnt, ist der Näherungssensor 50 auf einer eigenen, in den Figuren 4 und 5 nicht dargestellten Sensorplatine montiert. Das Vorsehen der räumlich von der Platine 71 getrennten Sensorplatine ermöglicht es auf einfache Weise, eine Ausführungsform der Vorrichtung 10₂ bereitzustellen, die einerseits berührungslos unter Verwendung des Näherungssensors 50 aktivierbar ist, aber andererseits nicht über die Platine 71 und folglich die hierauf montierten Sensoren, beispielsweise den Temperatursensor 58, verfügt. Eine derartige Ausführungsform der Vorrichtung 10₂ kann dann sinnvoll sein, wenn eine Dokumentation nicht notwendig ist.

Wie erwähnt, zeigt die Figur 5 die Vorrichtung 10₂ in einer zweiten Konfiguration. Vergleicht man die Figuren 4 und 5, so stellt man fest, dass die Pump- und Dosiereinheit 20 in der zweiten Konfiguration vom Gehäuse 12 entfernt worden und ein anderes Behältnis 18 in den Hohlraum 14 eingesetzt worden ist. Dieses Behältnis 18 weist eine eigene Pump- und Dosiereinheit 66 auf, welche in diesem Fall unlösbar mit dem Behältnis verbunden ist. Diese Pump- und Dosiereinheit 66 umfasst auch eine eigene Abgabeöffnung 68.

Das Behältnis 18, welches in Figur 5 in den Hohlraum 14 eingesetzt ist, weist zudem eine deutlich geringere Höhe auf als das Behältnis, das in Figur 4 in den Hohlraum eingesetzt worden ist. Um zu gewährleisten, dass die Pump- und Dosiereinheit 66 in Wechselwirkung mit der Antriebseinheit 48 kommen kann und dass die Abgabeöffnung 68 so angeordnet ist, dass das abgegebene Fluid auch den Hohlraum 14 verlassen kann, ist ein Stützkörper 70 auf die Auflagefläche 59 aufgelegt, auf den sich das Behältnis 18 abstützt. Alternativ kann der Stützkörper auch in das Gehäuse 12 eingehängt werden. Hierdurch wird die geringere Höhe des Behältnisses 18 ausgeglichen.

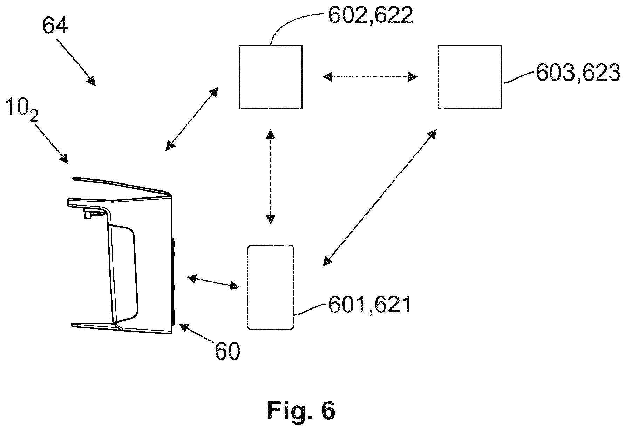

Im Folgenden wird Bezug auf Figur 6 genommen, in welcher ein System 64 zum Überwachen der in den Figuren 4 und 5 gezeigten Vorrichtung 10₂ anhand einer prinzipiellen Darstellung gezeigt ist. Das System 64 umfasst die Vorrichtung 10₂, die bereits erwähnte erste externe Einheit 621, eine zweite externe Einheit 622 und eine dritte externe Einheit 623, wobei die dritte externe Einheit 623 nur fakultativ vorhanden sein muss. Die Anzahl der Vorrichtungen 10₂, der ersten externen Einheiten 621, der zweiten externen Einheiten 622 und der dritten externen Einheiten 623 kann beliebig gewählt werden.

Wie erwähnt, kann die erste externe Einheit 621 als ein Barcode ausgeführt sein, der von der Sende- und Empfangseinheit 60 der Vorrichtung 10₁ ausgelesen werden kann. Folglich ist es nicht zwangsläufig notwendig, dass die erste externe Einheit 621 über eine erste Sende- und Empfangseinheit 601 verfügt, wobei dies beispielsweise dann der Fall ist, wenn die erste externe Einheit 621 als ein Smartphone oder als ein Transponder ausgeführt ist.

Die zweite externe Einheit 622 kann beispielsweise ein Server eines Krankenhauses, einer Arztpraxis oder einer Fabrik sein, der beispielsweise über ein WLAN-Netz mit der Vorrichtung 10₂ kommunizieren kann und folglich eine zweite Sende- und Empfangseinheit 602 aufweist. Die zweite externe Einheit 622 kann Daten mit der dritten externen Einheit 623 austauschen, welche ebenfalls als ein Server ausgestaltet sein kann, der mit einer Vielzahl von zweiten externen Einheiten 622 unter Verwendung einer dritten Sende- und Empfangseinheit 603 kommuniziert. Je nach Ausgestaltung der ersten externen Einheit 621, der zweiten externen Einheit 622 und der dritten externen Einheit 623 kann die dritte externe Einheit 633 auch mit der ersten externen Einheit 621 kommunizieren.

Das System 64 kann beispielsweise auf folgende Weisen betrieben werden: Wie erwähnt, können der Füllstand des Fluids im Behältnis 18 mit dem Füllstandssensor 56 und die Temperatur des Fluids im Behältnis 18 und die Temperatur in der unmittelbaren Umgebung der Vorrichtung 10₁ mittels des Temperatursensors 58 überwacht werden. Für den Fall, dass der Füllstand des Fluids im Behältnis 18 einen bestimmten Wert unterschreitet, kann von der zweiten externen Einheit 622 ein entsprechendes Signal erzeugt und an eine bestimmte Person übermittelt werden. Diese Person kann beispielsweise der Besitzer der ersten externen Einheit 621 sein, welche als ein Smartphone ausgebildet ist. Diese Person kann nun den Austausch des Behältnisses veranlassen, so dass vermieden wird, dass das sich in der Vorrichtung 10₂ befindende Behältnis 18 vollständig entleert wird und somit die Vorrichtung 10₂ nicht bestimmungsgemäß verwendet werden kann.

Wenn die Temperatur, welche vom Temperatursensor 58 erfasst wird, über eine bestimmte Zeitspanne auf einen bestimmten Wert steigt, so kann dies ein Hinweis auf eine Verkeimung des Fluids sein. Wiederum erzeugt die zweite externe Einheit 622 ein entsprechendes Signal und sendet es an die erste externe Einheit 621, so dass die verantwortliche Person ebenfalls den Austausch des sich in der Vorrichtung 10₂ befindenden Behältnisses veranlassen kann. Steigt die vom Temperatursensor 58 gemessene Temperatur innerhalb einer sehr kurzen Zeit auf einen ungewöhnlich hohen Wert an, so kann dies als einen Hinweis auf einen Brand gedeutet werden. Die zweite externe Einheit 622 kann nun einen Feueralarm auslösen. Da der Standort der Vorrichtung 10₂, dessen Temperatursensor 58 den Temperaturanstieg registriert hat, bekannt ist, kann der Brandherd lokalisiert werden.

Aufgrund der Tatsache, dass mit der ersten externen Einheit personenbezogene Daten mit der Vorrichtung 10₂ ausgetauscht werden, ist es möglich festzustellen, welche Personen zu welchem Zeitpunkt die Vorrichtung 10₂ verwendet hat. Die entsprechende Information wird an die zweite externe Einheit 622 übermittelt, wo eine entsprechende Dokumentation vorgenommen wird, die beispielsweise an das Gesundheitsamt oder die Berufsgenossenschaft übermittelt werden kann. Für den Fall, dass die betreffende Person die Vorrichtung 10₂ nicht wie vorgesehen verwendet hat, kann eine entsprechende Mitteilung an diese Person ausgegeben werden.

Für den Fall, dass festgestellt wird, dass eine Person sich mit einem bestimmten Krankheitserreger infiziert hat, wird diese Information in der dritten externen Einheit 623 gespeichert. Hat die Person vor dem Zeitpunkt, an dem die Infektion festgestellt worden ist, die Vorrichtung 10₂ verwendet, so kann ein entsprechendes Signal an erzeugt werden, welches auf die Notwendigkeit hinweist, die Vorrichtung 10₂ beispielsweise durch Autoklavieren so schnell wie möglich zu desinfizieren. Des Weiteren können weitere Personen, welche die Vorrichtung 10₂ kurz nach der infizierten Person benutzt haben, informiert werden, um entsprechende Gegenmaßnahmen einleiten zu können.

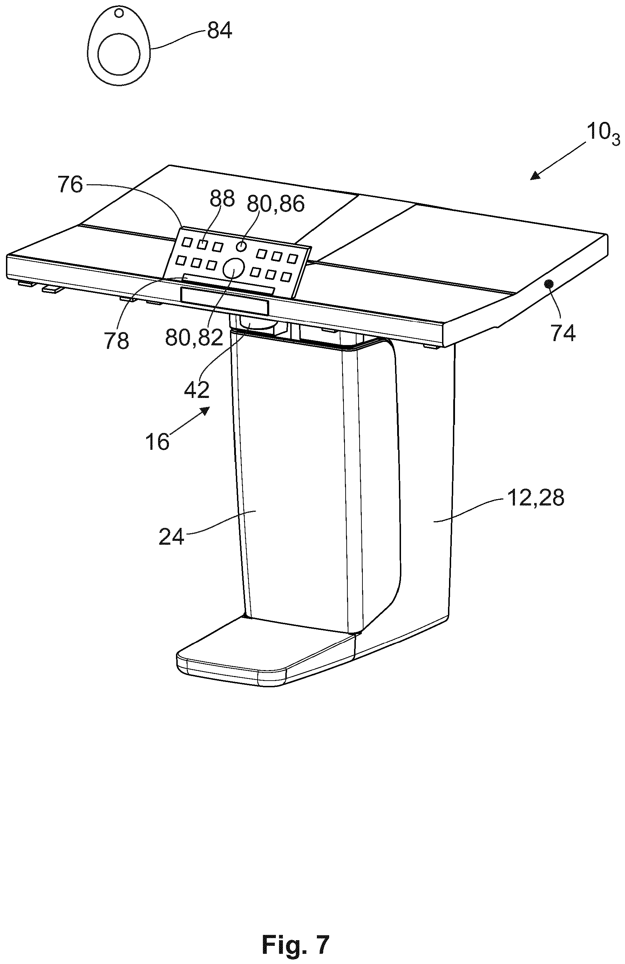

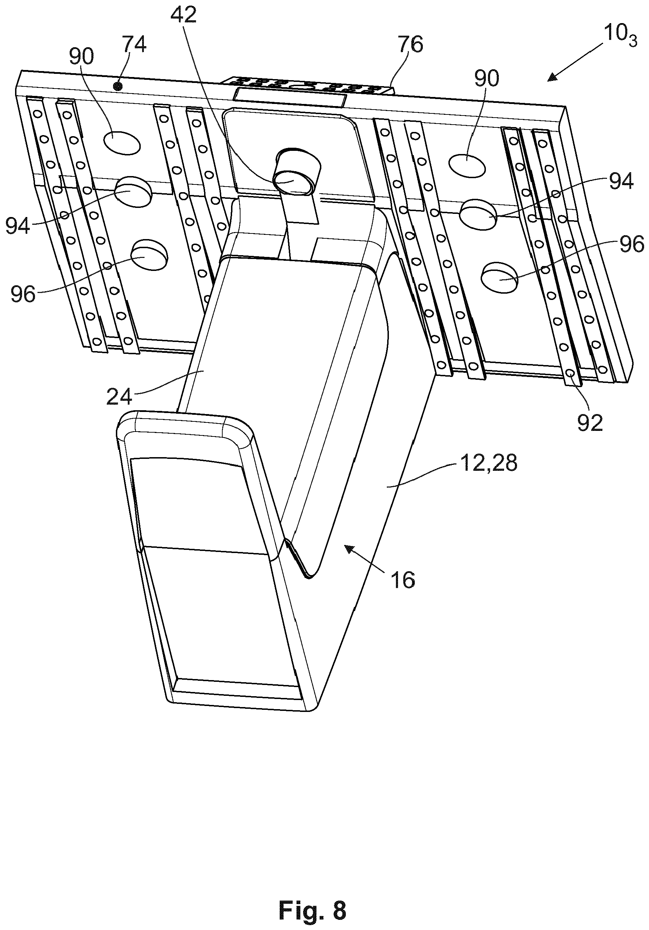

In den Figuren 7 und 8 ist eine dritte Ausführungsform der vorschlagsgemäßen Vorrichtung 10₃ anhand von perspektivischen Darstellungen gezeigt. Die Vorrichtung 10₃ nach der dritten Ausführungsform umfasst alle Merkmale der zweiten Ausführungsform der Vorrichtung 10₂ und baut folglich auf dieser auf. Insbesondere weist die Vorrichtung 10₃ nach der dritten Ausführungsform die in den Figuren 7 und 8 nicht sichtbare Antriebseinheit 48 auf. Es wird im Folgenden nur auf die zusätzlichen Merkmale Bezug genommen.

Die Vorrichtung 10₃ nach dem dritten Ausführungsbeispiel weist eine Deckenplatte 74 auf, welche mit dem Gehäuse 12 beispielsweise mittels einer Rastverbindung verbunden ist. Die Deckenplatte 74 kann aber auch als ein integraler Bestandteil des Gehäuses 12 gefertigt sein. In Figur 7 schaut man auf die Oberseite der Deckenplatte 74. Die Deckenplatte 74 ragt nach links und nach rechts über das Gehäuse 12 hinaus. An der Deckenplatte 74 ist ein Display 76 befestigt, mit welchem nutzerbezogene Daten und vorrichtungsbezogene Daten oder sonstige Daten angezeigt werden können. Hierzu weist das Display 76 einen Anzeigeabschnitt 78 auf.

Ferner ist im Display 76 eine Benutzererkennungseinrichtung 80 angeordnet, welche eine Ausleseeinheit 82 zum Auslesen eines hier nur prinzipiell dargestellten RFID-Transponders 84 umfasst. Alternativ oder kumulativ umfasst die Benutzererkennungseinrichtung 80 eine zweite Kamera 86, die ebenfalls im Display 76 angeordnet ist.

Das Display 76 umfasst zudem eine Anzahl von Tastenfeldern 88, in diesem Fall zwölf Tastenfelder 88, wobei jedes der Tastenfelder 88 einer bestimmten, mit einem Desinfektionsvorgang in Verbindung stehenden Tätigkeit zugeordnet ist. Hierauf wird später noch genauer eingegangen.

Figur 8 zeigt die Vorrichtung 10₃ gemäß der dritten Ausführungsform anhand einer zweiten perspektivischen Darstellung, in welcher man auf die Unterseite der Deckenplatte 74 schauen kann. An der Unterseite der Deckenplatte 74 sind zwei Ultraschallsensoren 90 angeordnet, welche Ultraschall abgeben und reflektierten Ultraschall erfassen können.

Weiterhin ist an der Unterseite der Deckenplatte 74 eine Vielzahl von Lichtquellen 92 bandförmig angeordnet, welche ultraviolette Strahlung aussenden können. Diese Lichtquellen 92 sind im dargestellten Ausführungsbeispiel als LED-Streifen ausgebildet, die sich von der vorderen Kante bis zur hinteren Kante der Deckenplatte 74 erstrecken.

Darüber hinaus sind zwei UV-Sensoren 94 vorgesehen, die ebenfalls an der Unterseite der Deckenplatte 74 befestigt sind und reflektierte UV-Strahlen erfassen können.

Ferner sind zwei erste Kameras 96 an der Unterseite der Deckenplatte 74 angeordnet. Sowohl die Ultraschallsensoren 90 als auch die Lichtquellen 92, die UV-Sensoren 94 und die beiden ersten Kameras 96 wirken mit der hier nicht sichtbaren Überwachungseinheit 54 zusammen.

Die Vorrichtung 10₃ nach dem dritten Ausführungsbeispiel kann auf folgende Weise betrieben werden.

Ein Benutzer, beispielsweise ein Mitarbeiter eines Krankenhauses, der sich die Hände desinfizieren will, hält einen ihm zugeordneten RFID-Transponder 84 vor die Ausleseeinheit 82. Je nach Ausgestaltung der Vorrichtung 10₃ kann stattdessen die zweite Kamera 86 beispielsweise die Gesichtszüge des Mitarbeiters erfassen. Die Ausleseeinheit 82 und die zweite Kamera 86 können auch parallel zueinander betrieben werden, um eine Redundanz zu schaffen. Ziel ist es, den Mitarbeiter zu identifizieren. Ist der Mitarbeiter erfolgreich identifiziert, kann auf dem Anzeigeabschnitt 78 des Displays 76 eine entsprechende Information ausgegeben werden. Bei einer erfolglosen Identifizierung kann eine entsprechende Fehlermeldung ausgegeben werden.

Für den Fall einer erfolgreichen Identifizierung kann der Mitarbeiter nun eines der Tastenfelder 88 anwählen, welches derjenigen Tätigkeit zugeordnet ist, welche der Mitarbeiter gerade durchführen will, beispielsweise einen Katheterwechsel. Anschließend hält der Mitarbeiter seine Hände unter die erste Kamera 96 und/oder unter den Ultraschallsensor 90. Die Vorrichtung 10₃ prüft nun, ob der Mitarbeiter Schmuck wie einen Ring trägt oder Kunstnägel hat. Ist dies der Fall, wird eine entsprechende Meldung ausgegeben, wonach gegen die geltenden Vorschriften verstoßen worden ist. Hat der Mitarbeiter keine Kunstnägel und trägt keinen Schmuck, so wird eine entsprechende OK-Meldung ausgegeben und der Mitarbeiter kann nun seine Hände unter die Abgabeöffnung 42 bewegen. Diese Bewegung wird vom in Figur 8 nicht dargestellten Bewegungssensor 50 registriert (siehe beispielsweise Figuren 4 und 5), infolgedessen die Antriebseinheit 48 aktiviert und eine bestimmte Menge des Fluids abgegeben wird. Die Menge des abgegebenen Fluids kann dabei abhängig von der gewählten Tätigkeit sein. Dem Fluid ist ein fluoreszierendes Additiv zugesetzt, welches in Wechselwirkung mit den von den Lichtquellen 92 ausgesendeten UV-Strahlen sichtbar wird. Nachdem der Mitarbeiter das Fluid in seine Handoberflächen einmassiert hat, kann er selbst bereits erkennen, ob die gesamte Handoberfläche vom Fluid benetzt worden ist oder ob Bereiche fehlen. Hierzu hält der Benutzer die Hände unter die Lichtquellen 92, welche zeitgleich zur oder kurz nach der Aktivierung der Antriebseinheit 48 eingeschaltet worden sind. Mittels der UV-Sensoren 94 und/oder mittels der ersten Kameras 96 kann mittels der Vorrichtung 10₃ geprüft werden, ob die gesamte Handoberfläche vom Fluid benetzt worden ist oder ob Bereiche fehlen. Ist die gesamte Handoberfläche vom Fluid benetzt, wird eine weitere OK-Meldung ausgegeben, womit die Handdesinfektion erfolgreich abgeschlossen ist. Fehlen Bereiche, muss das Fluid erneut einmassiert oder zusätzliches Fluid angefordert werden.

Sämtliche Daten, die während der Handdesinfektion von der Überwachungseinheit 54 der Vorrichtung 10₃ protokolliert worden sind, werden mittels der Sende- und Empfangseinheit 60 (vgl. Figuren 4 und 5) an die externen Einheiten 62 übermittelt. Die externen Einheiten 62 dokumentieren, dass der betreffende Mitarbeiter die betreffende Tätigkeit ausgeführt hat, nachdem er seine Hände vorschriftsmäßig desinfiziert hat. Die Information, dass die Handdesinfektion erfolgreich abgeschlossen worden ist, kann auch dazu verwendet werden, den betreffenden Mitarbeiter den Zugang zum Patienten zu gewähren. Hierzu können die externen Einheiten 62 mit einem Türöffner zusammenwirken, der die Tür zum Zimmer, in dem sich der Patient befindet, erst nach erfolgreich abgeschlossener Handdesinfektion öffnet.

Es kann vorgeschrieben sein, dass die Hände erneut desinfiziert werden müssen, wenn der Mitarbeiter die betreffende Tätigkeit abgeschlossen hat. Dann kann der oben beschriebene Vorgang erneut durchgeführt werden. Auch diese Handdesinfektion wird dokumentiert.

Je nach gewählter Tätigkeit und nach Ausgestaltung der Vorrichtung 10₃ können die oben beschriebenen Schritte zur Handdesinfektion auch in einer anderen Reihenfolge durchgeführt werden. Zudem können zusätzliche Schritte vorgesehen sein oder Schritte weggelassen werden.

### Bezugszeichenliste

- 10: Vorrichtung
- 10₁ - 10₃: Vorrichtung
- 12: Gehäuse
- 14: Hohlraum
- 16: Öffnung
- 18: Behältnis
- 20: Pump- und Dosiereinheit
- 22: Betätigungshebel
- 24: Verschlusskörper
- 26: Aufnahmeöffnung
- 28: Gehäuseaußenoberfläche
- 30: Pump- und Dosiereinheit-Außenoberfläche
- 32: Verschlusskörper-Außenoberfläche
- 33: antibakterielle Oberfläche
- 34: superhydrophobe Oberfläche
- 36: Pumpenstempel
- 38: erste Feder
- 40: Kanal
- 42: Abgabeöffnung
- 44: Saugrohr
- 45: Schlauch
- 46: Rückschlagventil
- 47: zweite Feder
- 48: Antriebseinheit
- 49: Befestigungseinrichtung
- 50: Bewegungssensor
- 51: Adaptereinheit
- 52: Ultraschallsensor, Näherungssensor
- 53₁, 53₂: rohrförmiger Abschnitt
- 54: Überwachungseinheit
- 55: Dichtkörper
- 56: Füllstandssensor
- 57: Öffnung
- 58: Temperatursensor
- 59: Auflagefläche
- 60: Sende- und Empfangseinheit
- 601 - 603: erste bis dritte Empfangseinheit
- 62: externe Einheit
- 621 - 623: erste bis dritte externe Einheit
- 64: System
- 66: eigene Pump- und Dosiereinheit
- 68: Abgabeöffnung
- 70: Stützkörper
- 71: Platine
- 72: weiterer Hohlraum
- 73: Batterie
- 74: Deckenplatte
- 75: Ethernet-Anschluss
- 76: Display
- 78: Anzeigeabschnitt
- 80: Benutzererkennungseinrichtung
- 82: Ausleseeinheit
- 84: RFID-Transponder
- 86: zweite Kamera
- 88: Tastenfeld
- 90: Ultraschallsensor
- 92: Lichtquellen
- 94: UV-Sensoren
- 96: erste Kamera

## Patentansprüche

1. Vorrichtung (10) zum Abgeben eines Fluids, insbesondere eines Reinigungs-, Pflege- oder Desinfektionsfluids für Hände, umfassend
- ein Gehäuse (12), welches
∘ einen Hohlraum (14) umschließt, in welchen ein Behältnis (20) zum Vorhalten des Fluids einbringbar ist, und
∘ eine Gehäuseaußenoberfläche (28) bildet,
- eine Pump- und Dosiereinheit (20),
∘ welche mit dem Gehäuse (12) verbindbar oder verbunden ist,
∘ welche mit dem Behältnis (20) lösbar verbindbar ist, und
∘ mit welcher das Fluid aus dem Behältnis (20) heraus gefördert und abgegeben werden kann,
- eine Überwachungseinheit (54) umfasst, mit welcher Vorrichtungsbezogene und/oder nutzerbezogene Daten erfasst und gespeichert werden können, und
- ein mit der Überwachungseinheit (54) zusammenwirkendes Display (76) zum Eingeben und Anzeigen der nutzerbezogenen Daten und/oder der vorrichtungsbezogenen Daten.

2. Vorrichtung (10) nach Anspruch 1,
**dadurch gekennzeichnet, dass** an der Gehäuseaußenoberfläche (28) zumindest ein Ultraschallsensor (90) angeordnet ist.

3. Vorrichtung (10) nach einem der Ansprüche 1 oder 2,
**dadurch gekennzeichnet, dass** an der Gehäuseaußenoberfläche (28) eine Anzahl von Lichtquellen (92) angeordnet ist, welche ultraviolette Strahlung emittieren.

4. Vorrichtung (10) nach Anspruch 3,
**dadurch gekennzeichnet, dass** die Vorrichtung zumindest einen UV-Sensor (94) zum Registrieren der von den Händen eines Benutzers reflektierten ultravioletten Strahlen umfasst.

5. Vorrichtung (10) nach einem der Ansprüche 3 oder 4,
**dadurch gekennzeichnet, dass** an der Gehäuseaußenoberfläche (28) zumindest eine erste Kamera (96) angeordnet ist.

6. Vorrichtung (10) nach einem der vorherigen Ansprüche,
**dadurch gekennzeichnet, dass** die Vorrichtung (10) eine Benutzererkennungseinrichtung (80) aufweist, mit welcher die Identität des Benutzers der Vorrichtung (10) festgestellt werden kann.

7. Vorrichtung (10) nach Anspruch 6,
**dadurch gekennzeichnet, dass** die Benutzererkennungseinrichtung (80) eine Ausleseeinheit (82) zum Auslesen eines RFID-Transponders (84) aufweist.

8. Vorrichtung (10) nach einem der Ansprüche 6 oder 7,
**dadurch gekennzeichnet, dass** die die Benutzererkennungseinrichtung (80) eine zweite Kamera (86) umfasst.

9. Vorrichtung (10) nach einem der Ansprüche 7 oder 8,
**dadurch gekennzeichnet, dass** die Ausleseeinheit (82) und/oder die zweite Kamera (86) im Display (76) angeordnet sind.

10. Vorrichtung (10) nach einem der vorherigen Ansprüche,
**dadurch gekennzeichnet, dass** im Display (76) eine Anzahl von Tastenfeldern (88) angeordnet ist, wobei jedes Tastenfeld (88) einer bestimmten, mit einem Desinfektionsvorgang in Verbindung stehenden Tätigkeit zugeordnet ist.

11. Vorrichtung (10) nach einem der vorherigen Ansprüche,
**dadurch gekennzeichnet, dass** die Vorrichtung (10) eine Sende- und Empfangseinheit (60) zum Datenaustausch mit externen Einheiten (62) umfasst.

12. Vorrichtung (10) nach einem der vorherigen Ansprüche,
**dadurch gekennzeichnet, dass** die Vorrichtung (10)
- einen Füllstandssensor (56) zum Bestimmen des Füllstands des Fluids im Hohlraum (18) und/oder
- einen Temperatursensor (58) zum Bestimmen der Temperatur des Fluids im Hohlraum (18) umfasst.

13. Vorrichtung (10) nach einem der vorherigen Ansprüche,
**dadurch gekennzeichnet, dass** die Antriebseinheit (48) mit einem berührungslos auslösbaren Bewegungssensor (50) aktivierbar ist und der Bewegungssensor (50) als ein Ultraschallsensor (52) oder als ein Näherungssensor (52) ausgestaltet ist.

14. Vorrichtung (10) nach einem der vorherigen Ansprüche,
**dadurch gekennzeichnet, dass** die Gehäuseaußenoberfläche (28) als eine antibakterielle Oberfläche (33) und/oder als eine superhydrophobe Oberfläche (34) ausgebildet ist.

15. System zum Überwachen einer Vorrichtung (10) nach den Ansprüchen 11 und 14, umfassend
- eine Vorrichtung (10) nach den Ansprüchen 11 bis 14,
- eine erste externe Einheit (621), auf welcher nutzerbezogene Daten gespeichert sind, die mittels der Sendeund Empfangseinheit (60) auslesbar sind, und
- eine zweite externe Einheit (622), auf welcher die nutzerbezogenen Daten und vorrichtungsbezogene Daten ablegbar und auswertbar sind, wobei die zweite externe Einheit (622) eine zweite Sende- und Empfangseinheit (602) zum Datenaustausch mit der Sende- und Empfangseinheit (60) der Vorrichtung (10) aufweist.

16. System nach Anspruch 15,
**dadurch gekennzeichnet, dass** die zweite Sende- und Empfangseinheit (602) derart eingerichtet ist, dass ein Datenaustausch mit einer dritten externen Einheit (623) ermöglicht wird.
